# EUROPEAN PATENT SPECIFICATION

(11) **EP 4 061 239 B1**
(45) Date of publication and mention of the grant of the patent: **28.10.2026**
(21) Application number: 20828187.3
(22) Date of filing: 23.11.2020
(51) Int. Cl.: A61B 17/00, A61B 17/34, A61B 5/00, A61B 5/07

(54) **INSERTABLE PHYSIOLOGICAL MONITOR INJECTOR TOOL**
EINFÜHRBARES PHYSIOLOGISCHES ÜBERWACHUNGSEINSPRITZWERKZEUG
OUTIL D'INJECTION DE MONITEUR PHYSIOLOGIQUE INSÉRABLE

(30) Priority: 21.11.2019 US 201962938793 P
(43) Date of publication of application: 28.09.2022
(73) Proprietor: Bardy Diagnostics, Inc., Seattle, WA 98104 (US)
(72) Inventor: BARDY, Gust H., Carnation, Washington 98014 (US); FLOYD, Jared, Ferndale, Washington 98248 (US); CHOI, John, Seattle, Washington 98104 (US); REDDY, Daniel L., Seattle, Washington 98125 (US); CRAN, Brian, Seattle, Washington 98177 (US)
(74) Representative: Reddie & Grose LLP
(86) International application number: PCT/US2020/061846
(87) International publication number: WO 2021/102426

(56) References cited:
- US-A- 4 846 793
- US-A1- 2004 199 140
- US-A1- 2014 324 067
- US-A1- 2016 235 346
- US-B1- 6 190 350

## Description

### TECHNICAL FIELD

This application relates in general to medical tools, and in particular to an insertable physiological monitor injector tool.

### BACKGROUND ART

Medical diagnosis includes evaluating patient physiology, which describes the vital functions of the patient's anatomical structure, that is, the living body and its organs. A patient's physiology is determined through medical diagnostic procedures that include performing medical tests and, when available, reviewing patient data that has been collected through monitoring, although the data should first be correlated to patient symptoms to be of relevant diagnostic value.

Sporadic conditions present a special challenge because diagnostic tests performed in a physician's office may prove ineffective if the sporadic condition fails to present while the test is being performed. Sporadic conditions may be due to chronic or acute cause and can include transient signs, such as erratic heartbeat, muscle or nerve spasms, or hypoglycemia (or hyperglycemia) that may be accompanied by discernable symptoms. The unpredictable nature of sporadic conditions often makes the capturing of physiological data a matter of good timing. If the sporadic condition fails to occur during the course of a medical test, no physiological data, and therefore no diagnostic insight, is obtained.

In response, physicians have turned to ambulatory monitoring, which utilizes sensors placed cutaneously on or implanted within a patient's body that are attached to a recorder to provide physiological data capture while the patient goes about daily life.

Ambulatory monitors that are either wholly implanted inside the patient's body or which use implanted sensors will generally provide cleaner physiological data relatively free of environmental noise and effects, especially when compared to data captured cutaneously. However, a tool or other means for performing implantation of the ambulatory monitor is required. Implantation can be invasive to some degree and carries more risk than cutaneous or external forms of ambulatory monitoring. Accordingly, such implantation means must be minimally invasive and ensure low risk of harm to a wearer of the monitor.

Current implantation tools include a tool described in U.S. Patent Application Publication No. 2018/0280056, to Austin, which includes a core having a longitudinal recess and a shell that protrudes from an end of the core that forms a compartment for a medical implant. A rod is arranged in the longitudinal recess and is connected to a grip member that encompasses the core. After the shell has been inserted into a patient, a user holds the grip member and the core retracts by sliding within the grip member to slide the rod for keeping position of the medical implant at the implantation side as the core retracts. Withdrawal of the core and shell from a patient depends on the user that pulls the core out of the incision site, which can create risk to a patient, such as if the user pulls the device out too fast or at an incorrect angle, potentially causing a larger incision to form or tissue damage.

Another implantation tool is described by U.S. Patent Application, Publication No. 2018/0168686, to Jin that includes an insertion housing having a passage and an obturator with a receptacle that is in communication with the passage of the housing. A distal end of the obturator is inserted through an incision on a patient and force is applied to the obturator to maintain the distal end within the patient as extended from the insertion housing. When in place, the obturator is withdrawn, allowing an implant to drop into the passage and the obturator is then moved forcing the implant to discharge from the tool. Thus, implant requires multiple steps for movement of the obturator, including withdrawing the obturator from an extended position, movement of the obturator back to the extended position to force the implant into the patient. Both injector tools referenced above are used for non-rechargeable battery powered devices and thus, fail to provide a means for recharging if the implant is packaged with the injector tool.

US6190350 discloses an implanter for inserting subcutaneous implants includes a handle for grasping the implanter during implantation, a hollow cannula receiving the implant, and a rod for releasing the implant from the cannula. The rod is longitudinally fixed within the handle while the cannula slides over the rod to release the implant. The cannula is moved over the rod by a sliding actuator mounted in a track of the handle. The actuator is locked in an extended position to prevent unintended release of the implant. Preferably, the implanter is a single-use device having a actuator locking feature preventing reuse.

US2004/199140 discloses a device for inserting implantable objects beneath the skin of a patient includes a handle for grasping the device and a base connected to the handle. The base comprises a post, a cannula, and a flexible actuator positioned in an angled track. The cannula is positioned coaxially around and is longitudinally slidable over the post from an extended position, where an implantable object is retained in the cannula, to a retracted position, where the implantable object is released from the cannula. A flexible actuator positioned on an angled track in the base is slidably engaged with a boss on the cannula and is used to move the cannula from an extended position to a retracted position to release the implantable object from the cannula; the actuator flexes between a locked and an unlocked position.

US 4 846 793 A describes a device for subcutaneous implantation of a plurality of solid, elongated medicinal pellets. The device includes a needle-like, hollow cannula within a barrel. The hub has a manually engageable knob thereon so that the hub and cannula may be easily moved with respect to the barrel with the thumb of one hand during the implantation process. An obturator extends from the rear of the barrel into the proximal end of the cannula. The obturator is composed of two or more sections which allow it to telescope during operation.

### DISCLOSURE OF THE INVENTION

The invention is defined by the independent claim. Preferred or optional features are set out in the dependent claims.

The invention, at least in preferred embodiments, provides an implant tool that safely and quickly injects a medical implant in a patient, while lowering risk of injury to the patient in a least invasive way, as well as simultaneously allowing for rechargeability of the medical implant that sits in the injector tool awaiting delivery to the patient. Devices may require recharging after packaging as they await implant into a patient, sometimes over months-long periods of time. Consequently, devices with rechargeable battery systems must use an injector technology that allows for recharging in situ. Accordingly, this injector accomplishes both safer insertion but also rechargeability while in the injector.

An insertable physiological monitor injector tool includes a handle within which an arbor and insertion tube are positioned. The insertion tube can have a hollow cylindrical form and be sized to house the arbor. A button can be positioned on an outer surface of the handle to retract the insertion tube when pressed or pulled back. During retraction of the insertion tube, the arbor remains stationary. The insertion tube is extended and preloaded with an insertable physiological monitor (IPM), such that one end of the IPM is adjacent to a distal end of the arbor. Examples of the IPM can include monitors for cardiac, oxygen, blood pressure, and glucose, as well as other types of monitors.

The insertion tube is inserted into the skin of a patient, at an angle, and the injector tool is rotated to lay flat along the skin to lift the skin up while performing a tunneling action. Once the insertion tube has reached a desired location for the IPM, the button is pressed to retract the insertion tube. The stationary arbor ensures the IPM remains in place, in the patient by preventing movement of the IPM with the insertion tube as the insertion tube retracts. Subsequently, injector tool is removed from the patient.

An embodiment provides an insertable physiological monitor injector tool. An elongated handle includes a recess formed along a longitudinal axis and has an opening on a distal end. An insertion tube has a hollow elongated shape that is movably positioned within the elongated handle, in the recess. A stationary arbor is affixed on a proximal end to a proximal end of the elongated handle and extends through the insertion tube when the insertion tube is in a retracted position. A tab is affixed to the insertion tube, wherein the tab can lock the insertion tube in an extended position.

A further embodiment provides an insertable physiological monitor injector tool having a structure through which a monitor housed in the structure is rechargeable.

Still other embodiments will become readily apparent to those skilled in the art from the following detailed description, wherein are described embodiments by way of illustrating the best mode contemplated. As will be realized, other and different embodiments are possible and the embodiments' several details are capable of modifications in various obvious respects, including time and clustering of events.

Accordingly, the drawings and detailed description are to be regarded as illustrative in nature and not as restrictive.

### DESCRIPTION OF THE DRAWINGS

FIGURE 1 is a block diagram showing, by way of example, a perspective view of an insertable physiological monitor injector tool.
FIGURE 2 is a block diagram showing, by way of example, an interior view of a cross section of a handle of the insertable physiological monitor injector tool of FIGURE 1.
FIGURE 3 is a block diagram showing, by way of example, a perspective view of an insertion tube of the insertable physiological monitor injector tool of FIGURE 1.
FIGURE 4 is a block diagram showing, by way of example, a perspective view of an incision tool.
FIGURE 5 is a block diagram showing, by way of example, a perspective view of a tunneller tool.
FIGURE 6 is block diagram showing, by way of example, a perspective view of an insertable physiological monitor injector tool loaded with an insertable physiological monitor.
FIGURE 7 is a block diagram showing, by way of example, a perspective view of an insertable physiological monitor injector tool in a partially retracted position.
FIGURE 8 is a block diagram showing, by way of example, a perspective view of an insertable physiological monitor injector tool in a fully retracted position.
FIGURE 9 is a block diagram showing, by way of example, a side view of an insertable physiological monitor injector tool in a fully retracted position.
FIGURE 10 is a block diagram showing, by way of example, a top view of an insertable physiological monitor injector tool in a fully retracted position.
FIGURE 11 is a block diagram showing, by way of example, a side view of an insertable physiological monitor injector tool in a fully retracted position.
FIGURE 12 is a block diagram showing, by way of example, a top view of a different embodiment of the insertable physiological monitor injector tool of FIGURE 1.
FIGURE 13 is a block diagram showing, by way of example, a side view of a distal end of an insertion tube of the embodiment of the insertable physiological monitor injector of FIGURE 12.
FIGURE 14 is a block diagram showing, by way of example, a perspective view of the insertable physiological monitor injector tool of FGURE 8.
FIGURE 15A is a block diagram showing, by way of example, a perspective view of an insertable physiological monitor injector tool with a channel.
FIGURE 15B is a block diagram showing, by way of example, a perspective view of an insertable physiological monitor injector tool with a tab in a channel stem.
FIGURE 15C is a block diagram showing, by way of example, a perspective view of an insertable physiological monitor injector tool with a tab on a distal end of a channel.
FIGURE 15D is a block diagram showing, by way of example, a perspective view of an insertable physiological monitor injector tool with a tab on a proximal end of a channel.
FIGURE 15E is a block diagram showing, by way of example, a side view of an insertable physiological monitor injector tool with a tab.

### BEST MODE FOR CARRYING OUT THE INVENTION

Implantable devices can provide patients with benefits, such as continuous monitoring of physiological signals, which can be helpful to detect sporadic events and facilitate diagnosis by a medical professional. However, injecting the implantable device can be invasive and come with some risk to the patient, such as infection or adverse reaction due to penetrating the patient's skin during injection. An injection tool that is minimally invasive, such as requiring a small incision, and is quick and efficient for implantation can help reduce risk to the patient.

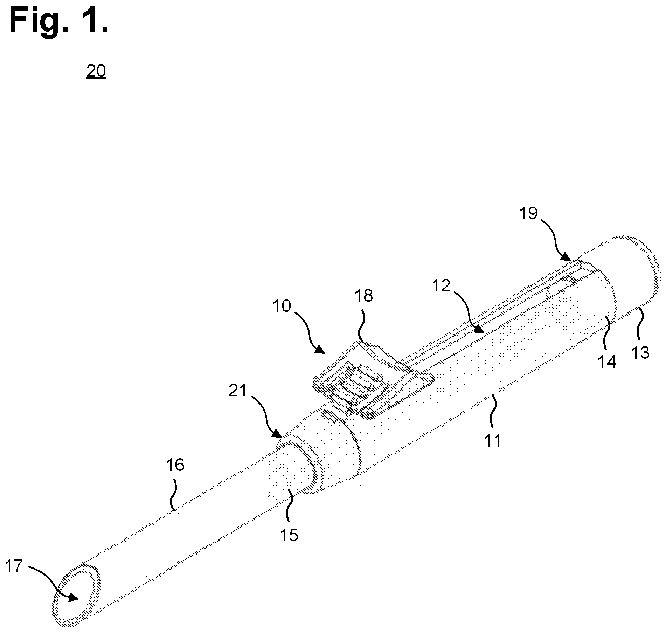

The minimally invasive incision tool can include an insertion tube, a handle, and an arbor. FIGURE 1 is a block diagram showing, by way of example, a perspective view 10 of an insertable physiological monitor injector tool 10. The injector tool 10 can include a handle 11 with a recess formed in an interior of the handle, along a longitudinal axis. The handle 11 can have a cylindrical semi-circular or rectangular shape, as well as other types of shapes. In one embodiment, a shape of the handle can be based on a shape of an implantable physiological monitor (IPM) to be injected into a patient using the injection tool. A proximal end 19 of the handle can be enclosed, while a distal end 21 of the handle can be open to allow access to the recess.

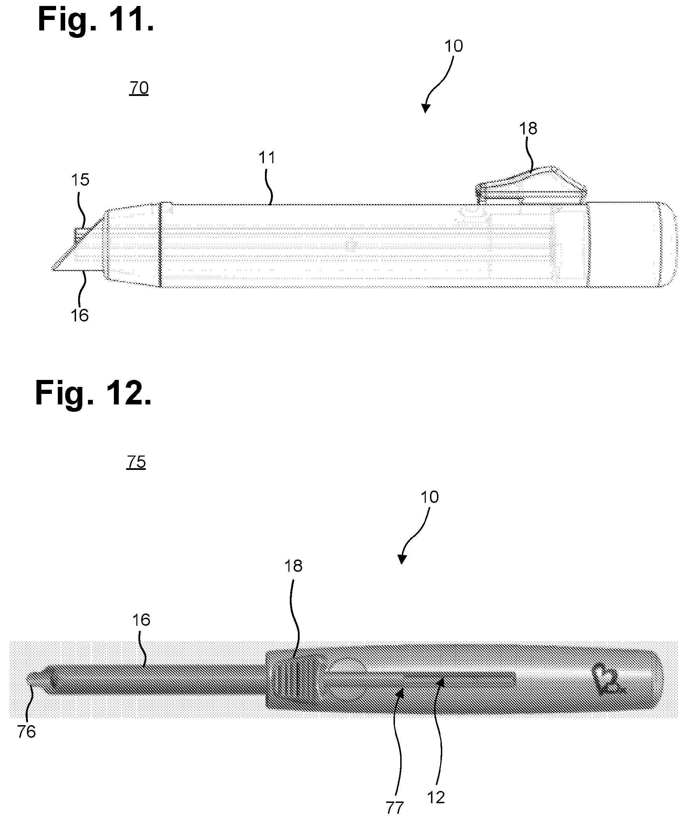

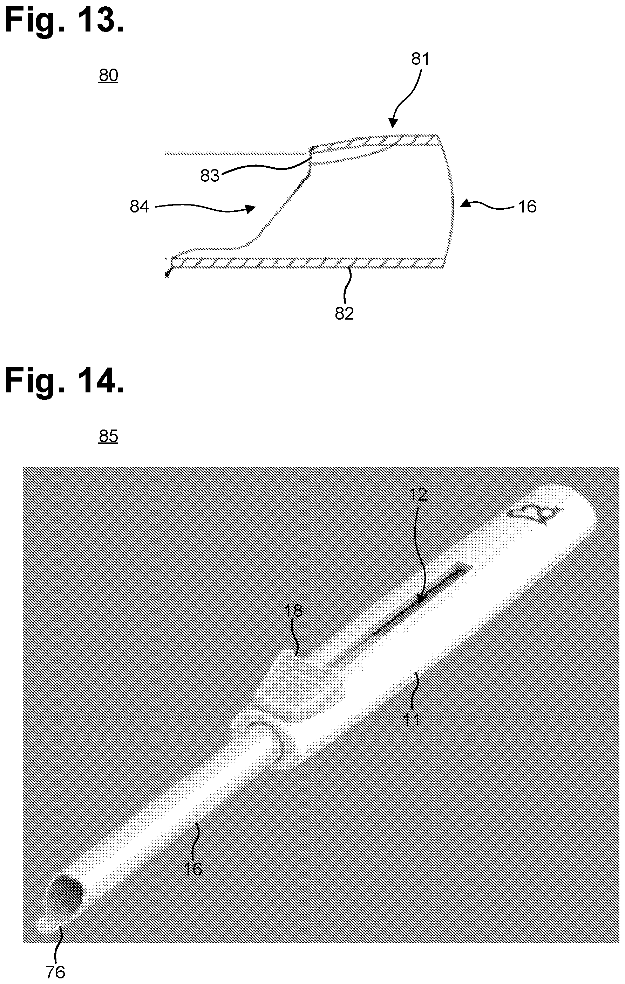

An insertion tube 16, which can have a different shape or the same shape as the handle, is provided in the recess of the handle. The insertion tube 16 can have a hollow interior 17 and be sized to fit in the recess 12 of the handle. At a minimum, a length of the insertion tube 16 should extend just beyond a distal end 21 of the handle via the opening. A distal end of the insertion tube can include a beveled edge or a straight edge, and can be sharpened or unsharpened. In a further embodiment, a tip can be attached to the distal end of the injector tube, as further described below with respect to the FIGURES 12 and 13.

A button or tab 18 is affixed to the insertion tube 16 to allow a user to move the insertion tube 16 within the recess. The button or tab 18 is positioned over an outer surface of the handle 11via a channel 22 formed by a cutout in the handle 11. The button can slide back and forth within the channel 22 to move the insertion tube 16 back and forth within the recess of the handle 11.

An arbor 15 fits within the insertion tube 16 and is affixed on one end to a proximal end 19 of the handle 11 or to a separate end piece 13 attached to the proximal end of the handle. In one embodiment, a proximal end of the arbor 15 is affixed to a support mount 14 that is then affixed to the proximal end of the handle or the separate end piece 13. The arbor 15 can be shaped as a circle, square, rectangle, oval, cross, or a different shape, and sized to fit within the insertion tube 16. A length of the arbor 15 can be dependent on a length of the handle and should extend slightly past the distal end of the handle 11. The arbor 15 remains in a fixed position, while the insertion tube 16 slides back and forth over the arbor 15.

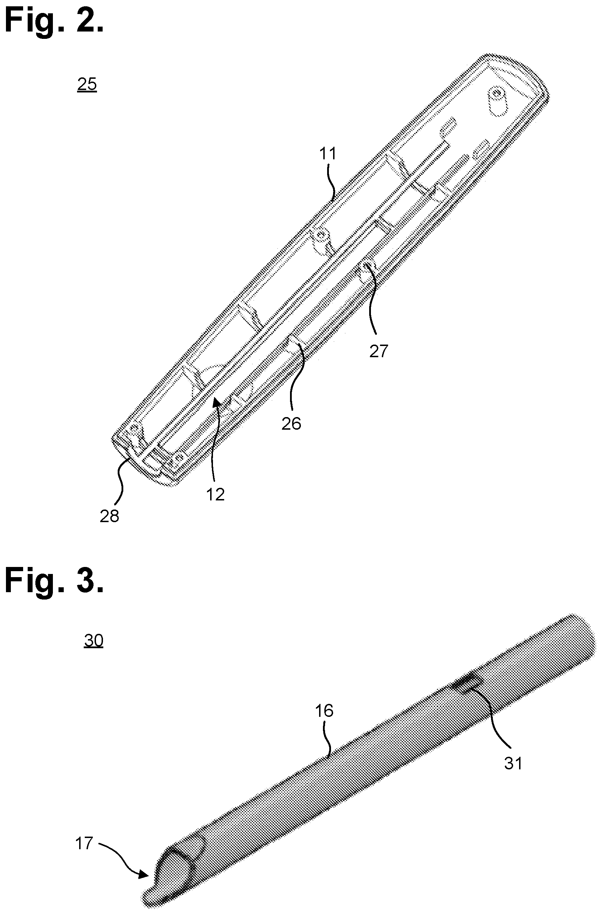

To ensure smooth movement of the insertion tube 16 within the handle 11, the handle can be conformed to fit securely around the insertion tube 16 with a slight space between an interior surface of the handle 11 and an exterior of the insertion tube 16. When the shape of the handle 11 differs from the insertion tube 16, an interior of the handle 11 can include guides to allow smooth movement of the insertion tube 16. FIGURE 2 is a block diagram showing, by way of example, an interior view 25 of a cross section of a handle of the insertable physiological monitor injector tool of FIGURE 1. The handle 11 can be formed from a single piece of material or from two or more pieces. The material can include metal, or stainless steel, as well as other types of conductive material. In an interior of the handle 11, guides 26 can be formed along the recess 12 to guide the insertion tube (not shown) along the longitudinal axis of the recess 12. The guides can be formed as tabs of material, such as plastic, metal, or stainless steel, that extend from an interior surface of the handle 11. The guides can extend around or partially around the insertion tube (not shown). One or more sets of guides can be provided in the handle and each set can include two guides, which are each formed on either side of the insertion tube.

When the handle is formed from multiple pieces of material, screw holes or other fastening mechanism 27 can be formed along an interior of the handle. Screw holes or other fastening mechanism located on another piece of the handle should correspond with the screw holes on a first piece to secure the different pieces of the handle 11 together. At least one end of the handle is open 28, including the proximal end 21 of the handle to allow movement of the insertion tube (not shown) in and out of the handle 11.

The insertion tube slides within the recess via a button or tab. FIGURE 3 is a block diagram showing, by way of example, a perspective view 30 of an insertion tube 16. The insertion tube 16 can be formed as a cylindrical tube with a hollow interior. However, other shapes are possible. Both ends of the insertion tube 16 can be open 17 to allow movement of the insertion tube 16 over the arbor (not shown). A cutout 31 is formed in a surface of the insertion tube 16 to affix to the button (not shown). For example, the button (not shown) can include a tab that is inserted into and secured in the cutout to allow a user to slide the button and thus, the insertion tube 16.

The insertion tube 16 can be made from material, including metal, stainless steel, plastic, or other types of material. When made from plastic, the IPM can be recharged while inserted in the insertable physiological monitor injector tool 10, as described below in further detail. A length of the insertion tube can be dependent on a location of an implantation site at which the IPM will be implanted. For example, an implantable cardiac monitor can be implanted between the skin and ribs of a wearer. Accordingly, the insertion tube 16 must be long enough to extend past the skin of a patient, but not longer than a distance between the ribs and the skin. Further, a shape of the insertion tube can be dependent on the IPM to be inserted in a wearer. For example, the insertion tube can be cylindrical when the IPM is cylindrical.

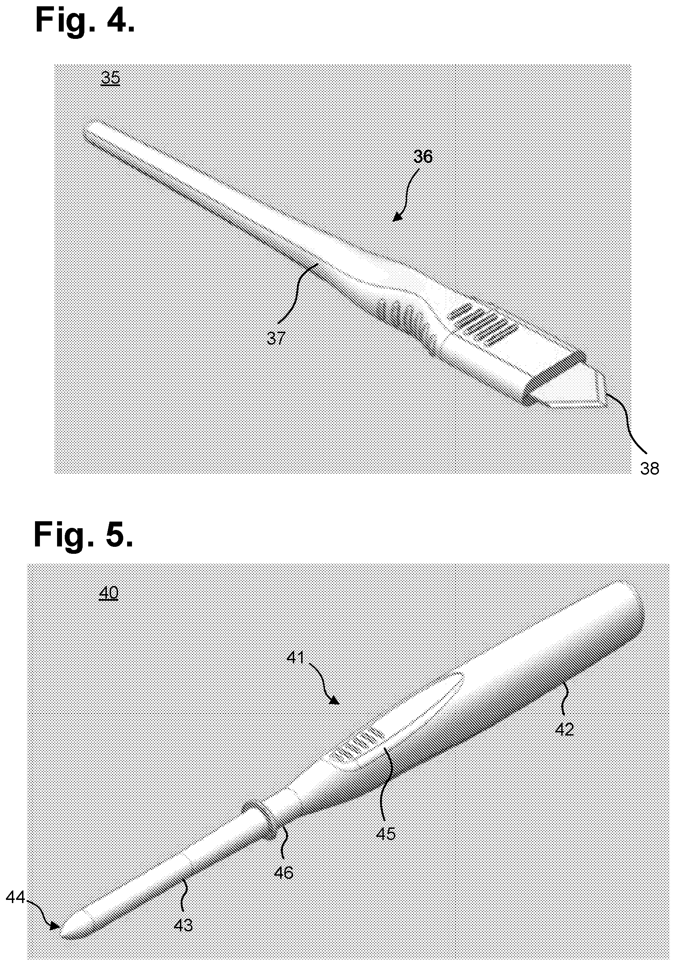

Implantation of the IPM can utilize an incision tool, tunneller tool, and the insertable physiological monitor injector tool. FIGURE 4 is a block diagram showing, by way of example, a perspective view 35 of an incision tool 36. The incision tool 36 includes a handle 37 and a blade 38. The blade can be in the shape of a triangle, rectangle, or another shape. When shaped as a triangle, a base of the triangle can be affixed to a distal end of the handle 37 and an apex of the triangle facing outward from the handle 37. The incision tool 36 is used to make an incision in the skin of a patient after the incision area has been cleaned and sanitized.

After an incision has been made, a tunneller tool is used to clear a path in the patient's tissue for later insertion of the insertable physiological monitor injector tool. FIGURE 5 is a block diagram showing, by way of example, a perspective view 40 of a tunneller tool 41. The tunneller tool 41 includes a handle 42, a stopper or depth meter 46, and a rod 43. The handle 42 includes a grip 45 for a user when inserting the tunneller into a patient. The stopper or depth meter 46 is affixed on a distal end of the handle to provide a particular length of the rod which is to be inserted into the patient, while the rod 43 extends from the stopper. The stopper or depth meter 46 can be stationary or adjustable to adjust a length of the rod for different depth insertions in the patient. A distal end of the rod can include a tip 44 that can be sharpened or unsharpened. A length of the rod 43 can be dependent on a location of the IPM to be placed.

The tip 44 of the rod is inserted into the incision made by the incision tool. Once inserted, the user pushes the rod further into the patient via the handle 42 to clear a path in the tissue for placement of the IPM. The user can push the tunneller tool 41 into the patient until the stopper touches or is adjacent to the skin of the patient. Subsequently, the user removes the tunneller tool 41 from the patient.

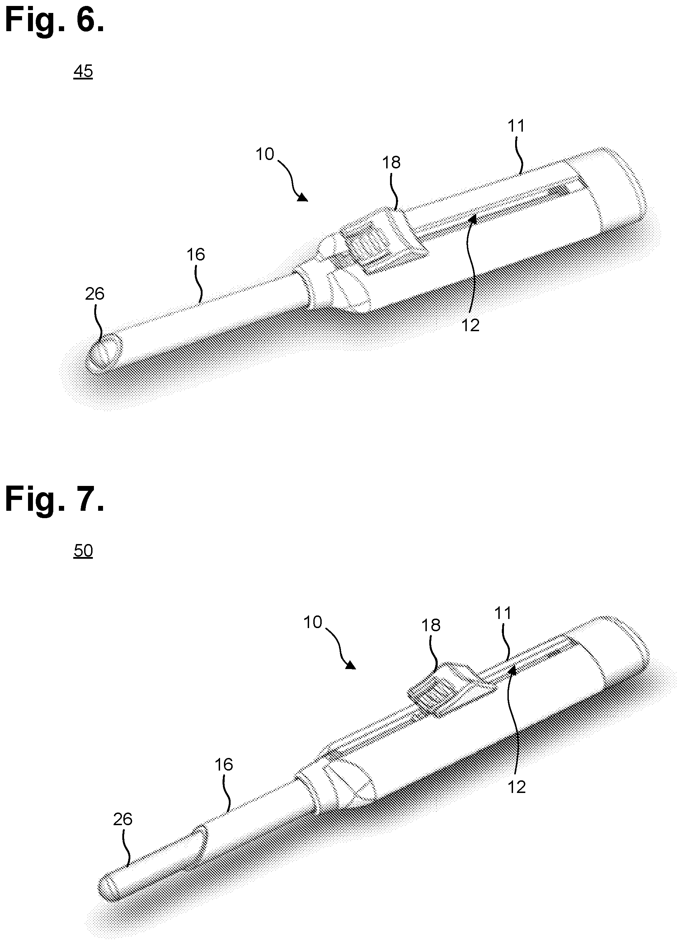

Once the incision has been made and a path is cleared for placement of the IPM, the insertable physiological monitor injector tool can be inserted into the patient. FIGURE 6 is block diagram showing, by way of example, a perspective view 45 of an insertable physiological monitor injector tool loaded with an IPM. For injection, the insertion tube 16 can be in an extended position, outside of the recess of the handle 11. An ICM 26 is placed within the insertion tube 16 and is adjacent on a proximal end to the arbor (not shown).

A distal end of the extended insertion tube 16 is inserted into the incision on the patient and a user moves the insertable physiological monitor injector tool into the tissue of the patient using the path formed by the tunneller tool. In one embodiment, the user can press down on or distally against the button 18 to prevent the insertion tube 16 from retracting towards a proximal end of the handle 11 via the recess 12. However, in a further embodiment, the button can be in a locked position to prevent retraction of the insertion tube 16.

Once a desired location has been reached by the distal end of the insertion tube 16, FIGURE 7 is a block diagram showing, by way of example, a perspective view 50 of an insertable physiological monitor injector tool 10 in a partially retracted position. The insertable physiological monitor injector tool 10 can be inserted into the patient until the distal end of the housing touches or is adjunct to an outward facing surface of the patient's skin. Once reached, the button 18 can be pressed or released from a spring loaded or locked position, depending on a type of safety mechanism, and slowly moved along the channel 12, towards the proximal end of the handle 11. As the button is moved proximally, the insertion tube 16 slides over the IPM 26 which is stationary and begins to get exposed to the patient's tissue as the insertion tube retracts.

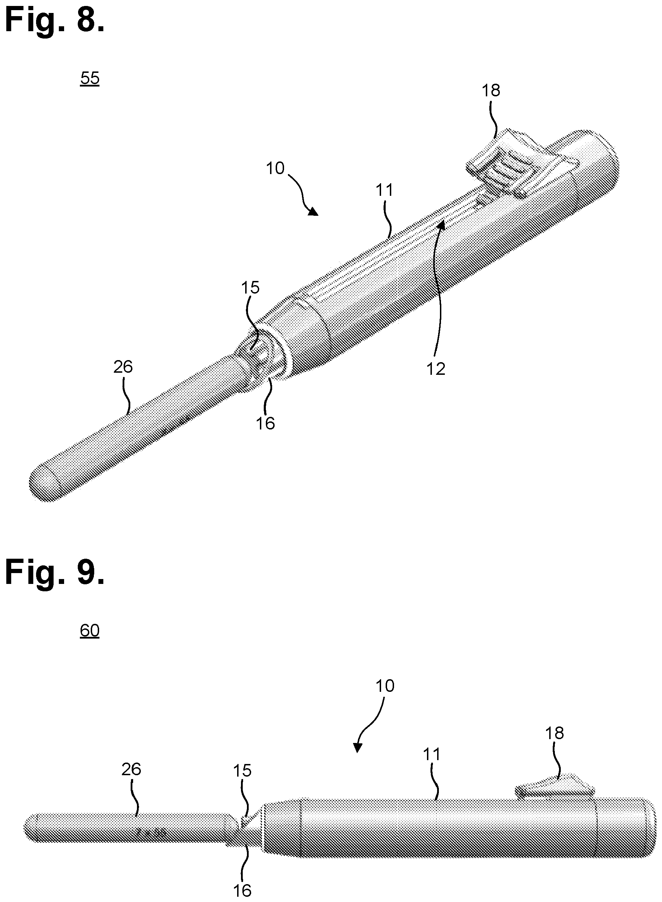

Once the insertion tube has been fully retracted, the IPM is positioned outside of the insertion tube and fully in the tissue of the patient. FIGURE 8 is a block diagram showing, by way of example, a perspective view 55 of an insertable physiological monitor injector tool 10 in a fully retracted position. To obtain the retracted position, the user should slide the button 18 the length of the channel 22, formed in the handle 11, to move the insertion tube 16 along the recess 12 into an interior of the handle 11. As the insertion tube is retracted, more of the IPM 26 is revealed and introduced to the tissue of the patient. The stationary arbor 15 within the insertable tube is adjacent to a proximal end of the IPM 26 and prevents the IPM 26 from retracting with the insertable tube 16. Thus, the IPM 26 remains in the patient's tissue and the insertable physiological monitor injector tool 10 is moved away from the patient after the insertion tube 16 has been fully retracted.

FIGURE 9 is a block diagram showing, by way of example, a side view 60 of an insertable physiological monitor injector 10 in a fully retracted position. In the fully retracted position, the button 18 is at the proximal end of the handle 11, the insertion tube 16 is fully retracted and a portion of the insertion tube 16 may extend slightly past the distal end of the handle. The arbor remains stationary and also extends slightly past the distal end of the handle, while the IPM 26 is no longer housed by the insertion tube 16 and is left in the tissue of the patient at the desired implantation site.

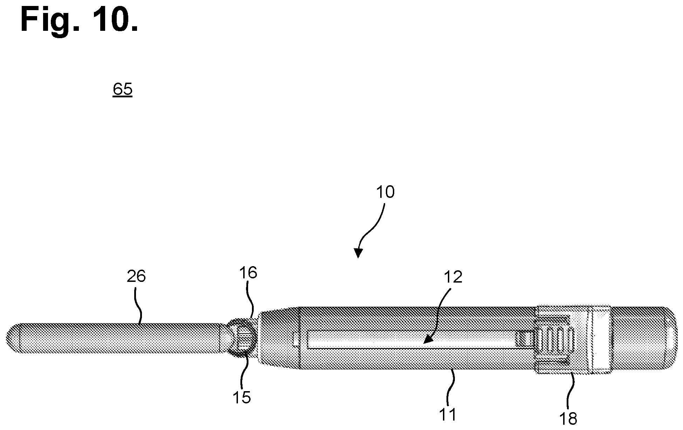

FIGURE 10 is a block diagram showing, by way of example, a top view 65 of an insertable physiological monitor injector tool 10 in a fully retracted position. When the insertion tube 16 is fully retracted, the button 18 is on a proximal end of the handle and the IPM 26 is no longer in the insertable physiological monitor injector tool 10. The IPM 26 is positioned in the tissue of the patient and remains as the insertable physiological monitor injector tool is removed from the patient. The arbor 15 prevents the IPM 26 from retracting with the insertion tube 16 along the recess of the handle during retraction and removal of the insertable physiological monitor injector tool 10.

When in the fully retracted position, the arbor is positioned within the insertion tube and the insertion tube is within the recess of the handle. FIGURE 11 is a block diagram showing, by way of example, a side view 70 of an insertable physiological monitor injector tool 10 in a fully retracted position. In the fully retracted position, the button 18 is on the proximal end of the insertable physiological monitor injector tool 10. The insertion tube 16 is positioned in the recess of the handle and the arbor 15 is positioned within the insertion tube 16.

The above description of the insertable physiological monitor injector tool can have different embodiments. For example, FIGURE 12 is a block diagram showing, by way of example, a top view 75 of a different embodiment of the insertable physiological monitor injector 10 of FIGURE 1. A tip 76 can be formed on at least a portion of the distal end of the insertion tube 16. The tip 76 can be heat formed to prevent the IPM (not shown) from falling out of the insertion tube 16 during insertion into the patient. FIGURE 13 is a block diagram showing, by way of example, a side view 80 of a distal end of an insertion tube16 of the embodiment of the insertable physiological monitor injector of FIGURE 12. A distal end 81 of the insertion tube 16 can include a tip 83 for preventing an IPM placed in an interior 84 of the insertion tube 16 from falling out, especially when the injector tool is angled for insertion into a patient.

In one embodiment, the tip 81 can be formed on an upper surface of the insertion tube, nearest to the button. The tip 81 can be formed inward, into the hollow interior 84, to produce interference between the insertion tube and the ICM. For example, the material 82 of the insertion tube 16 can bend downward on the distal tip, into the hollow interior 84. However, bending the tip 83 too far into an interior of the insertion tube can prevent release of the IPM when the insertion tube retracts. Accordingly, the tip 83 should be formed to prevent the IPM from falling out, but allow the insertion tube to retract over the IPM, which is stationary due to the arbor (not shown).

In a further embodiment, the tip can include an additional piece of material affixed to an upper interior surface of the insertion tube material 82. The additional material can extend downward into the hollow interior of the insertion tube to prevent the IPM from falling out.

When the insertion tube 16 is fully extended, the button 18 is on a distal end of the handle 11 and the recess 12 in the handle 11 can be visible. Also, a proximal end 77 of the insertion tube 16 can be visible through the channel. However, when in a fully retracted position, the recess 12 may not be visible since the outer surface of the insertion tube 16 can block a view of the recess 12 via the channel 22.

FIGURE 14 is a block diagram showing, by way of example, a perspective view of the insertable physiological monitor injector of FGURE 12. In an extended position, the insertion tube 16 extends outward from the handle 11 and the button 18 is located on a distal end of the handle 11. The tip 76 extends from a distal end of the insertion tube 16 on a bottom surface of the opening. However, in a further embodiment, the tip can extend from a top surface or fully around the opening of the insertion tube 16 on the distal end. The tip can prevent the IPM (not shown) from moving outside of the insertion tube 16 when the insertable physiological monitor injector tool 10 is inserted into a patient.

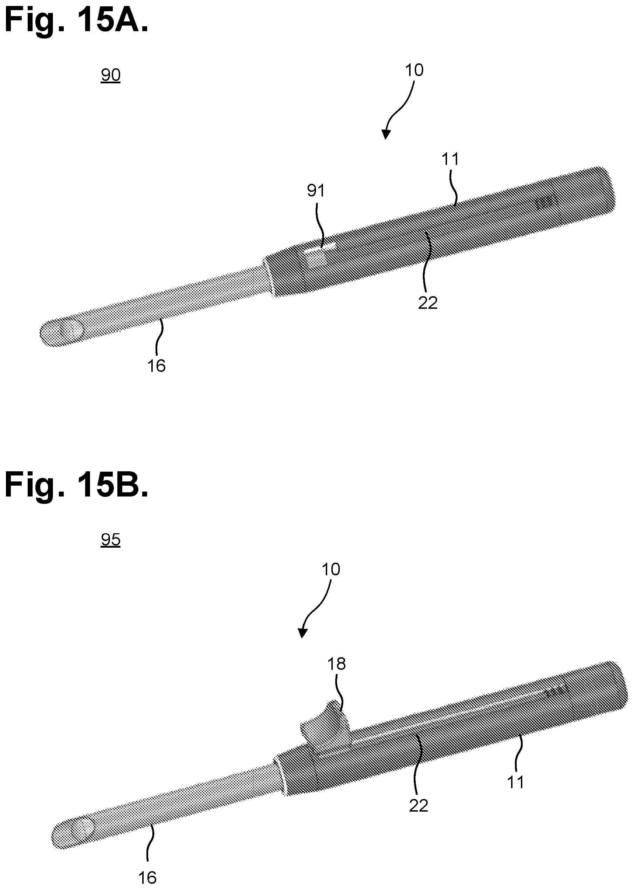

FIGURES 15A-E are block diagrams showing, by way of example, a different embodiment of the insertable physiological monitor injector of FIGURE 1. FIGURE 15A is a block diagram showing, by way of example, a perspective view 90 of an insertable physiological monitor injector tool 10 with a channel 22. The housing 11 of the insertable physiological monitor injector tool 10 has a channel 22 formed within a surface, such as a top surface. The channel 22 can run along a longitudinal axis of the housing 11 and include a stem 91, which is formed on a distal end of the housing and extends outwards, away from the channel along the longitudinal axis. The stem 91 can function to lock the insertion tube 16 in an extended position via a tab (not shown).

FIGURE 15B is a block diagram showing, by way of example, a perspective view 95 of an insertable physiological monitor injector tool 10 with a tab 18 in a channel stem. A tab, such as a button 18 can be in a locked position when located in the stem (not shown) of the channel 22 formed in the handle 11. Specifically, when in the stem, the button 18 is prevented from moving distally or proximally along the handle, which prevents movement of the insertion tube 16. Thus, when the button or tab 18 is locked, the insertion tube 16 is locked in an extended position.

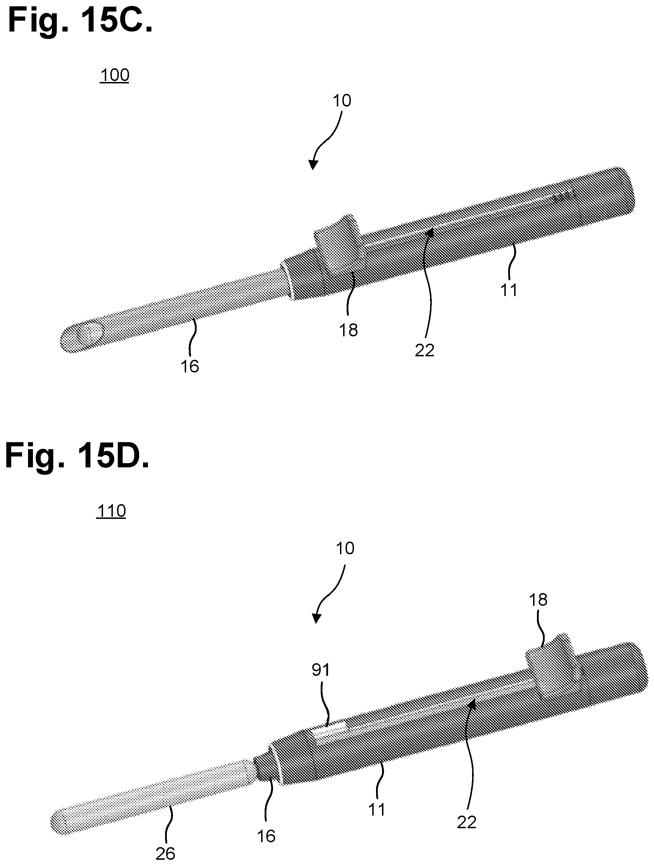

FIGURE 15C is a block diagram showing, by way of example, a perspective view 100 of an insertable physiological monitor injector tool 10 with a tab 18 on a distal end of a channel 22. To unlock the insertion tube 16, of the insertable physiological monitor injector tool 10, from an extended position, a user slides a tab, such as a button 18 out of the stem (not shown) and into the channel 22. Specifically, a user moves the button or tab 18 across the longitudinal axis of the handle 11 and towards the channel 22.

FIGURE 15D is a block diagram showing, by way of example, a perspective view 110 of an insertable physiological monitor injector tool 10 with a tab 18 on a proximal end of a channel 22. To retract the insertion tube 16, a user slides the tab, such as a button 18, away from the channel stem 91, towards a proximal end of the handle 11, along the channel 22. When the button 18 is on the proximal end, the insertion tube 16 moves into the recess of the handle and totally clears or mostly clears the IPM 26, leaving the IPM implanted in the tissue of a patient.

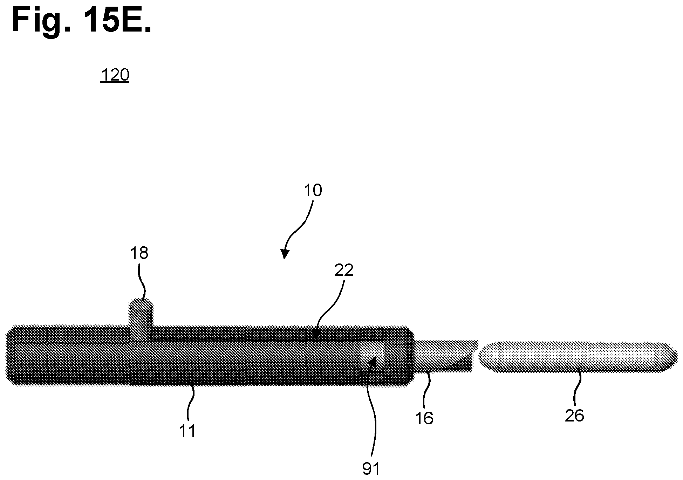

FIGURE 15E is a block diagram showing, by way of example, a side view 120 of an insertable physiological monitor injector tool 10 with a tab 18. The tab can include a button, knob, pin, or other type of device that is capable of attachment to the insertion tube 16 and movement by the user. At a minimum the tab should be movable from a locked position in a channel stem 91, into a channel 22, and then along the channel 22. As the tab 18 is moved proximally along the channel 22, the insertion tube retracts into an interior of the housing 11, leaving the IPM 26 in the tissue of a patient.

In one embodiment, the IPM can be packaged with the insertable physiological monitor injector tool, such as by placing the IPM in the insertion tube. The injection tube is in the extended position to provide room in which the IPM can be placed on a distal end of the arbor, a portion of which is also inside the insertion tube in the extended position. Based on how much time passes from packaging of the IPM with the injector tool to injection of the IPM in a patient, battery power of the IPM may run low. Ideally, the IPM should be fully charged prior to insertion into the patient to ensure that all functions of the IPM are working. When the insertion tube is made from a conductive material that allows electromagnetic waves or energy to pass, the battery of the IPM can be recharged while remaining in the insertable physiological monitor injector tool, prior to placement in the patient. The material of the insertion tube can include metal or other types of conductive material.

In one embodiment, the battery can be inductively charged via a wireless device that is placed over sterile packaging of the insertable physiological monitor injector tool. The wireless charging device can include a puck or wand or other type of device capable of wireless charging. The device wirelessly charges the battery until the battery is full. Subsequently, the insertable physiological monitor injector tool is removed from the package and used to insert the IPM in a patient.

The insertable physiological monitor injector tool can be constructed by building an elongated handle with a recess formed along a longitudinal axis in an interior of the handle. An insertion tube having a hollow elongated shape can be positioned within the recess of the elongated handle. One or more sets of guides are formed on an interior surface of the handle to surround and guide at least a portion of the insertion tube in the recess. In one embodiment, a notch can be formed on a proximal end of the insertion tube, which is moveable within the recess. A stationary arbor is placed within the insertion tube and affixed to a proximal end of the elongated handle, in the interior. When the insertion tube is in a retracted position, the stationary arbor extends through the insertion tube. A tab, such as a button or knob is affixed to the insertion tube via the notch. The tab can be used to lock the insertion tube in an extended position. An implantable physiological monitor is placed within the insertion tube when the insertion tube is in the extended position. A channel is formed within a portion of the handle, on the proximal end, to allow movement of the button to retract the insertion tube from the extended position to a retracted position.

The invention is defined by the appended claims.

## Claims

1. An insertable physiological monitor injector tool (10), comprising:
an elongated handle (11) comprising a substantially cylindrical housing within which a recess (12) is formed along a longitudinal axis and comprising an opening on a distal end;
an insertion tube (16) comprising a hollow elongated interior (84) that is movably positioned within the elongated handle (11), in the recess (12), wherein the insertion tube comprises a beveled end at a distal end of the insertion tube (16), wherein the beveled end comprises a tip (83) that bends inwards into the hollow elongated interior (84) of the insertion tube (16) to secure an implantable physiological monitor within the insertion tube (16) prior to implantation, wherein the insertion tube is made from a conductive material such that the implantable physiological monitor is inductively chargeable within the insertion tube (16);
a stationary arbor (15) affixed on a proximal end to a proximal end of the elongated handle (11) and extends through the insertion tube (16) when the insertion tube (16) is in a retracted position; and
a tab (18) directly affixed to an outer surface of the insertion tube (16) configured to move with respect to the stationary arbor, wherein the tab (18) is configured to lock the insertion tube (16) in an extended position.

2. An insertable physiological monitor injector tool (10) according to Claim 1, further comprising:
an implantable physiological monitor (26) placed within the insertion tube (16) when the insertion tube (16) is in the extended position.

3. An insertable physiological monitor injector tool (10) according to Claim 1, wherein a channel (22) is formed within a portion of the handle (11), on the proximal end, to allow movement of the tab (18).

4. An insertable physiological monitor tool (10) according to Claim 3, wherein the insertion tube (16) is configured to retract into the handle (11) via the recess (12) upon movement of the tab (18) in the channel (22) towards a proximal end of the handle (11).

5. An insertable physiological monitor injector tool (10) according to Claim 1, wherein the distal end of the insertion tube (16) is sharpened.

6. An insertable physiological monitor injector tool (10) according to Claim 1, further comprising:
one or more sets of guides formed on an interior surface of the elongated handle (11) and shaped to surround at least a portion of the insertion tube (16) in the recess (12).

7. An insertable physiological monitor injector tool (10) according to Claim 1, further comprising:
a notch (31) formed on a proximal end of the insertion tube (16).

8. An insertable physiological monitor injector tool (10) according to Claim 7, wherein
the tab (18) is affixed to the notch (31) of the insertion tube (16).

9. The insertable physiological monitor injector tool (10) according to claim 1, wherein the insertion tube (16) is configured to accept the implantable physiological monitor when the insertion tube (16) is in the extended position.

10. An insertable physiological monitor injector tool system comprising the injector tool (10) according to Claim 1 and further comprising:
an incision tool (36) comprising a handle (37) and a blade (38) affixed to a distal end of the handle (37).

11. An insertable physiological monitor injector tool system comprising the injector tool (10) according to Claim 10 and further comprising:
a tunneller tool (41) comprising a handle (42) and a rod (43) affixed to a distal end of the handle (42).

12. The insertable physiological monitor injector tool system according to any of claims 10 or 11,
comprising a charging device to recharge a battery of the insertable physiological monitor.

## Patentansprüche

1. Einführbares physiologisches Überwachungseinspritzwerkzeug (10), umfassend:
einen länglichen Griff (11), umfassend ein im Wesentlichen zylindrisches Gehäuse, in dem eine Aussparung (12) entlang einer Längsachse gebildet ist und umfassend eine Öffnung an einem distalen Ende;
einen Einführschlauch (16), umfassend einen hohlen länglichen Innenraum (84), der beweglich in dem länglichen Griff (11) in der Aussparung (12) positioniert ist, wobei der Einführschlauch ein abgeschrägtes Ende an einem distalen Ende des Einführschlauches (16) umfasst, wobei das abgeschrägte Ende eine Spitze (83) umfasst, die sich nach innen in den hohlen länglichen Innenraum (84) des Einführschlauches (16) biegt, um ein implantierbares physiologisches Überwachungsgerät vor dem Implantieren im Einführschlauch (16) zu sichern, wobei der Einführschlauch aus einem leitfähigen Material gefertigt ist, sodass das implantierbare physiologische Überwachungsgerät in dem Einführschlauch (16) induktiv aufladbar ist;
eine stationäre Spindel (15), die mit einem proximalen Ende an einem proximalen Ende des länglichen Griffs (11) fixiert ist und sich durch den Einführschlauch (16) erstreckt, wenn sich der Einführschlauch (16) in einer zurückgezogenen Position befindet; und
eine Lasche (18), die direkt an einer Außenoberfläche des Einführschlauches (16) fixiert ist, die ausgebildet ist, um sich in Bezug zur stationären Spindel zu bewegen, wobei die Lasche (18) ausgebildet ist, um den Einführschlauch (16) in einer ausgefahrenen Position zu verriegeln.

2. Einführbares physiologisches Überwachungseinspritzwerkzeug (10) nach Anspruch 1, weiter umfassend:
ein implantierbares physiologisches Überwachungsgerät (26), das in den Einführschlauch (16) platziert wird, wenn sich der Einführschlauch (16) in der ausgefahrenen Position befindet.

3. Einführbares physiologisches Überwachungseinspritzwerkzeug (10) nach Anspruch 1, wobei ein Kanal (22) innerhalb eines Abschnitts des Griffs (11) am proximalen Ende gebildet ist, um eine Bewegung der Lasche (18) zu ermöglichen.

4. Einführbares physiologisches Überwachungswerkzeug (10) nach Anspruch 3, wobei der Einführschlauch (16) ausgebildet ist, um sich bei Bewegung der Lasche (18) im Kanal (22) in Richtung eines proximalen Endes des Griffs (11) über die Aussparung (12) in den Griff (11) zurückzuziehen.

5. Einführbares physiologisches Überwachungseinspritzwerkzeug (10) nach Anspruch 1, wobei das distale Ende des Einführschlauches (16) geschärft ist.

6. Einführbares physiologisches Überwachungseinspritzwerkzeug (10) nach Anspruch 1, weiter umfassend:
einen oder mehrere Sätze von Führungen, die auf einer Innenoberfläche des länglichen Griffs (11) gebildet und geformt sind, um mindestens einen Abschnitt des Einführschlauches (16) in der Aussparung (12) zu umgeben.

7. Einführbares physiologisches Überwachungseinspritzwerkzeug (10) nach Anspruch 1, weiter umfassend:
eine Kerbe (31), die an einem proximalen Ende des Einführschlauches (16) gebildet ist.

8. Einführbares physiologisches Überwachungseinspritzwerkzeug (10) nach Anspruch 7, wobei die Lasche (18) an der Kerbe (31) des Einführschlauches (16) fixiert ist.

9. Einführbares physiologisches Überwachungseinspritzwerkzeug (10) nach Anspruch 1, wobei der Einführschlauch (16) ausgebildet ist, um das implantierbare physiologische Überwachungsgerät aufzunehmen, wenn sich der Einführschlauch (16) in der ausgefahrenen Position befindet.

10. Einführbares physiologisches Überwachungseinspritzwerkzeug, umfassend das Einspritzwerkzeug (10) nach Anspruch 1 und weiter umfassend:
ein Inzisionswerkzeug (36), umfassend einen Griff (37) und eine an einem distalen Ende des Griffs (37) fixierte Klinge (38).

11. System für ein physiologisches Überwachungseinspritzwerkzeug, umfassend das Einspritzwerkzeug (10) nach Anspruch 10 und weiter umfassend:
ein Tunnelungswerkzeug (41), umfassend einen Griff (42) und einen an einem distalen Ende des Griffs (42) fixierten Stab (43).

12. System für ein physiologisches Überwachungseinspritzwerkzeug entsprechend einem der Patentansprüche 10 oder 11, umfassend eine Ladevorrichtung, um eine Batterie des einführbaren physiologischen Überwachungsgeräts zu laden.

## Revendications

1. Outil injecteur (10) de moniteur physiologique insérable, comprenant :
une poignée allongée (11) comprenant un logement sensiblement cylindrique dans lequel un évidement (12) est formé le long d'un axe longitudinal et comprenant une ouverture sur une extrémité distale ;
un tube d'insertion (16) comprenant un intérieur allongé creux (84) qui est positionné de manière mobile au sein de la poignée allongée (11), dans l'évidement (12), dans lequel le tube d'insertion comprend une extrémité biseautée au niveau d'une extrémité distale du tube d'insertion (16), dans lequel l'extrémité biseautée comprend une pointe (83) qui est cintrée vers l'intérieur dans l'intérieur allongé creux (84) du tube d'insertion (16) pour arrimer un moniteur physiologique implantable au sein du tube d'insertion (16) avant l'implantation, dans lequel le tube d'insertion est constitué d'un matériau conducteur de sorte que le moniteur physiologique implantable peut être chargé par induction au sein du tube d'insertion (16) ;
un arbre fixe (15) fixé sur une extrémité proximale à une extrémité proximale de la poignée allongée (11) et qui s'étend à travers le tube d'insertion (16) lorsque le tube d'insertion (16) est dans une position rétractée ; et
une languette (18) fixée directement à une surface externe du tube d'insertion (16) configurée pour se déplacer par rapport à l'arbre fixe, dans lequel la languette (18) est configurée pour verrouiller le tube d'insertion (16) dans une position étendue.

2. Outil injecteur (10) de moniteur physiologique insérable selon la revendication 1, comprenant en outre :
un moniteur physiologique implantable (26) placé au sein du tube d'insertion (16) lorsque le tube d'insertion (16) est dans la position étendue.

3. Outil injecteur (10) de moniteur physiologique insérable selon la revendication 1, dans lequel un canal (22) est formé au sein d'une portion de la poignée (11), sur l'extrémité proximale, pour permettre un mouvement de la languette (18).

4. Outil (10) de moniteur physiologique insérable selon la revendication 3, dans lequel le tube d'insertion (16) est configuré pour se rétracter dans la poignée (11) via l'évidement (12) lors du mouvement de la languette (18) dans le canal (22) vers une extrémité proximale de la poignée (11).

5. Outil injecteur (10) de moniteur physiologique insérable selon la revendication 1, dans lequel l'extrémité distale du tube d'insertion (16) est pointue.

6. Outil injecteur (10) de moniteur physiologique insérable selon la revendication 1, comprenant en outre :
un ou plusieurs ensembles de guides formés sur une surface intérieure de la poignée allongée (11) et modelés pour entourer au moins une portion du tube d'insertion (16) dans l'évidement (12).

7. Outil injecteur (10) de moniteur physiologique insérable selon la revendication 1, comprenant en outre :
une encoche (31) formée sur une extrémité proximale du tube d'insertion (16).

8. Outil injecteur (10) de moniteur physiologique insérable selon la revendication 7, dans lequel la languette (18) est fixée à l'encoche (31) du tube d'insertion (16).

9. Outil injecteur (10) de moniteur physiologique insérable selon la revendication 1, dans lequel le tube d'insertion (16) est configuré pour accepter le moniteur physiologique implantable lorsque le tube d'insertion (16) est dans la position étendue.

10. Système d'outil injecteur de moniteur physiologique insérable comprenant l'outil injecteur (10) selon la revendication 1 et comprenant en outre :
un outil d'incision (36) comprenant une poignée (37) et une lame (38) fixée à une extrémité distale de la poignée (37).

11. Système d'outil injecteur de moniteur physiologique insérable comprenant l'outil injecteur (10) selon la revendication 10 et comprenant en outre :
un outil tunneliseur (41) comprenant une poignée (42) et une tige (43) fixée à une extrémité distale de la poignée (42).

12. Système d'outil injecteur de moniteur physiologique insérable selon l'une quelconque des revendications 10 ou 11, comprenant un dispositif de charge pour recharger une batterie du moniteur physiologique insérable.
